(19) Europäisches Patentamt
European Patent Office
Office européen des brevets

(11) Publication number: **0 603 800 A1**

(12) **EUROPEAN PATENT APPLICATION**

(21) Application number: **93120540.5**

(51) Int. Cl.$^5$: **A61K 31/557**

(22) Date of filing: **20.12.93**

(30) Priority: **21.12.92 US 993586**

(43) Date of publication of application:
**29.06.94 Bulletin 94/26**

(84) Designated Contracting States:
**AT BE CH DE DK ES FR GB GR IE IT LI LU MC NL PT SE**

(71) Applicant: **ALCON LABORATORIES INC**
**6201 South Freeway**
**Ft. Worth, TX 76134(US)**

(72) Inventor: **DeSantis, Louis, Jr.**
**2316 Winton Terrace West**
**Fort Worth, Texas 76109(US)**
Inventor: **Sallee, Verney L.**
**304 Diamond Lane**
**Burleson, Texas 76028(US)**

(74) Representative: **Reinhard, Skuhra, Weise**
**Friedrichstrasse 31**
**D-80801 München (DE)**

(54) **Prostaglandin combinations in glaucoma therapy.**

(57) The invention discloses a topical ophthalmic composition for the treatment of glaucoma and ocular hypertension, said composition comprising a combination of particular prostaglandins of the F and E series.

EP 0 603 800 A1

## BACKGROUND OF THE INVENTION

This invention relates to the use of combinations of prostaglandins of the F and E series and their derivatives and analogues for the treatment of glaucoma and ocular hypertension. As used herein, the terms "prostaglandin" and "PG" shall refer to prostaglandins and derivatives and analogues thereof, except as otherwise indicated by context.

The structures of the naturally-occurring prostaglandins of the F and E series, of which the prostaglandins of the present invention are derivatives and/or analogues, are shown below:

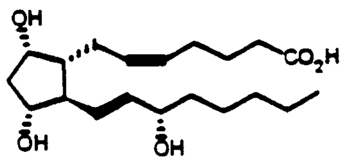

$PGF_{2\alpha}$

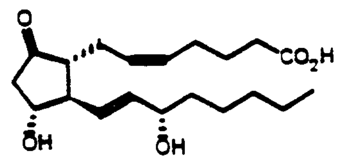

$PGE_2$

Naturally-occurring prostaglandins are known to lower intraocular pressure (IOP) after topical ocular instillation, but can cause an inflammatory response (hyperemia). Many synthetic prostaglandins have been observed to lower intraocular pressure, but most such compounds also produce the described inflammatory response. This has been found to be particularly true for prostaglandins of the E series.

Various methods have been used in attempting to overcome this well-known inflammatory response. Stjernschatz et al. (WO 90/02553) have striven to synthesize derivatives of naturally-occurring prostaglandins in order to design out selectively the inflammatory response while maintaining the IOP-lowering effect. Others, including Ueno et al. (EP 330 511 A2) and Wheeler (EP 435 682 A2) have tried complexing prostaglandins with various cyclodextrins.

## SUMMARY OF THE INVENTION

It has been unexpectedly discovered that co-administration of an E series prostaglandin and an F series prostaglandin in combination produces a greater reduction of IOP than the same dose of either type of compound given separately. In fact, as described in greater detail below, representative mixtures of the prostaglandins of the present invention produce a profound and long lasting IOP decrease. Administration of both types of prostaglandins in combination is apparently necessary to produce the desired IOP lowering effect for glaucoma therapy, while decreasing the likelihood of systemic side effects.

The extremely low dosage of the prostaglandin combinations of the present invention prevents or markedly decreases the local and/or systemic side effects seen with other glaucoma therapies -- especially those based on PG therapy. A dosage of a compound of formula (I) adequate to lower IOP produces local irritation and discomfort. The combination of a compound of formula (I) and a compound of formula (II) allows this dosage to be decreased by 90% or more, thereby eliminating or substantially reducing such irritation and discomfort.

Both $PGF_{2\alpha}$ and $PGE_2$ are naturally formed by the eye, and are normally present in aqueous humor as a combination. In addition, corneal tissue is capable of transforming exogenous $PGF_{2\alpha}$ into $PGE_2$ such that the $PGE_2$ concentration in aqueous humor is increased following topical ocular dosing with $PGF_{2\alpha}$. It is therefore reasonable to propose that the potent IOP lowering effect of the present PG combinations is somehow attributable to the combination of a compound of formula (I), with a compound of formula (II). The limited response to dosage with $PGF_{2\alpha}$ alone is consistent with this proposed explanation. Although the mechanism for the observed synergism is unknown, it is clear that dosage with an optimum combination of a compound of formula (I) and a compound of formula (II) will allow a more potent reduction of intraocular pressure without the side-effects produced by treatment with an adequate dose of a single component.

DETAILED DESCRIPTION OF THE INVENTION

The compounds of the present invention are known. See, for example, The Merck Index, 10th Edition (1983), which is incorporated herein by reference to the extent that it describes the preparation and known pharmacological profiles of $PGF_{2\alpha}$ and $PGE_2$. See also DE 2,223,365 (Bowler) and WO 90/02553 (Stjernschantz: et al.).

As used in this "Detailed Description of the Invention," the term "$PGF_{2\alpha}$" shall refer to the prostaglandins of formula (I), and the term "$PGE_2$" shall refer to the prostaglandins of formula (II).

Prostaglandins of the F series which are useful in the present invention have the general formula (I), shown below:

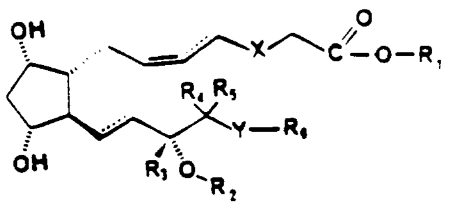

(I)

wherein:

| | |
|---|---|
| X and Y | can be the same or different, and are: $CH_2$ or O; |
| $R_1$ | is hydrogen, a cationic salt moiety, a pharmaceutically acceptable amine moiety or a pharmaceutically acceptable ester moiety derived from the corresponding alcohol; and |
| $R_2$ | is hydrogen or a pharmaceutically acceptable ester moiety derived from the corresponding carboxylic acid. |
| $R_3$, $R_4$ and $R_5$ | can be the same or different, and are: H or $CH_3$, with the proviso that if $R_3$ is $CH_3$, then $R_4$ and $R_5$ are H; and |
| $R_6$ | is: $C_{2-7}$ alkyl, thienyl or aryl, optionally substituted with one or more of the following: $C_{1-5}$ alkyl, trifluoromethyl, or a halogen; |

with the proviso that if Y is O, and $R_6$ is aryl, then the aryl group must contain at least one substituent.

The following are preferred compounds of formula (I): cloprostenol, fluprostenol, PhXA41, 16,16-dimethyl-$PGF_{2\alpha}$, 15-methyl-$PGF_{2\alpha}$, 16-(3,5-dichloro-phenoxy)-$PGF_{2\alpha}$, tiaprost, 17-phenyl-$PGF_{2\alpha}$, 17-*m*-chlorophenyl-$PGF_{2\alpha}$, 17-*m*-trifluoromethylphenyl-$PGF_{2\alpha}$,17-(3,5-dichlorophenyl)-$PGF_{2\alpha}$, and the 3-oxa- and 13,14-dihydro- derivatives of each, as appropriate. Structures of some of the preferred compounds are shown in the following Table 1. It is most preferred to use: cloprostenol, fluprostenol and PhXA41.

TABLE 1

| COMPOUND NAME | STRUCTURE |
|---|---|
| 1) PhXA41 | 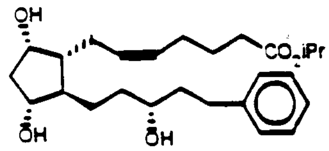 |
| 2) 16,16-dimethyl $PGF_{2\alpha}$ | 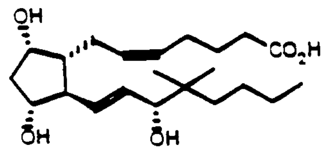 |
| 3) cloprostenol | 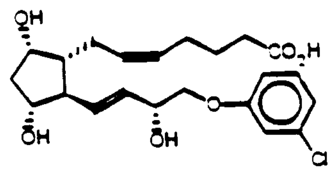 |
| 4) fluprostenol | 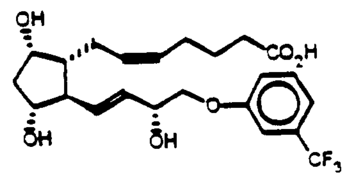 |
| 5) 13,14-dihydro-cloprostenol | 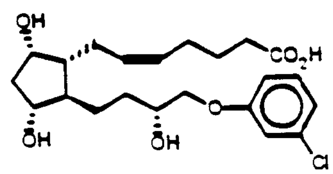 |
| 6) 3-oxa-cloprostenol | 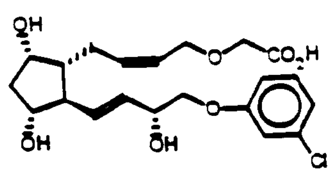 |
| 7) 15-methyl $PGF_{2\alpha}$ | 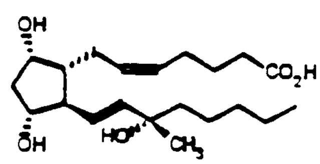 |
| 8) 15-acetyl-16,16-dimethyl-$PGF_{2\alpha}$ | 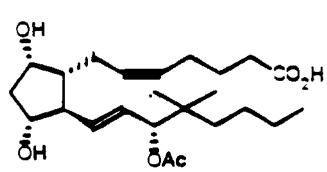 |

Prostaglandins of the E series which are useful in the present invention have the general formula (II), shown below:

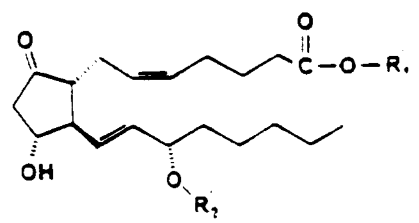

(II)

whererin:

$R'_1$ is hydrogen, a cationic salt moiety, a pharmaceutically acceptable amine moiety or a pharmaceutically acceptable ester moiety derived from the corresponding alcohol; and

$R'_2$ is hydrogen or a pharmaceutically acceptable ester moiety derived from the corresponding carboxylic acid.

As used herein, the term "pharmaceutically acceptable salts and esters" means esters and salts of these compounds which have the same general pharmacological properties as the acid form from which they are derived, and which are acceptable from a toxicity viewpoint. Specifically included by this term are salts and esters of the type disclosed in U.S. Patent No. 4,029,681 (June 19, 1977) and in U.S. Patent No. 4,288,616 (September 8, 1981), the disclosures of which are hereby incorporated in the present specification by reference. Thus, the compounds covered by the above general formulae include the free acid ($R'_1$, $R_1$ = H) and alcohol ($R'_2$, $R_2$ = H), alkali and alkaline earth metal salts (e.g., Na, K, Ca, and Mg), ammonium and amine salts, and esters ($R'_1$, $R_1$ = alkyl, or $R'_2$, $R_2$ = acyl). Preferred salts are those involving alkali and alkaline earth metal cations, particularly sodium and potassium, and amine salts, especially tris(hydroxymethyl)aminomethane salts. Preferred esters are $C_1$-$C_{12}$ alkyl esters, particularly straight or branched $C_1$-$C_6$ alkyl esters, especially methyl, ethyl, isopropyl, cyclopropyl, cyclopropyl methyl, butyl, cyclobutyl, isobutyl, butyl or pentyl.

Alkali metal salts and alkaline earth metal salts of the acid form of (I) and (II) may be formed conventionally. The alcohol and/or acid or salt may be subsequently esterified with the appropriate acid and/or alcohol, e.g., a $C_1$-$C_3$ alkyl alcohol, to yield the final ester product embodiment of (I) and (II) according to known procedures.

In a similar manner, other esterifications may be effected as is known in the art employing other low alkyl, cycloalkyl, cycloalkyalkyl, aryl, or aryalkyl alcohols and/or acids such as isopropanol, cyclopropanol, cyclopropylmethanol, or phenyl or benzyl alcohol. Since such esterification reactions are well known, they are not further described here.

Prostaglandins of formula (I) and formula (II) are combined in a molar ratio in the range of 0.1:1.0 to 1000:1, respectively. The preferred range is 4:1 to 20:1. Most preferred is a molar ratio of about 10:1.

The combinations of compounds of formulae (I) and (II) are useful in lowering intraocular pressure and thus are useful in the treatment of glaucoma. As compared with therapeutically effective dosages of the individual components, the combinations produce significantly fewer unwanted side effects such as marked vasoconstriction or vasodilation of the vessels of the sclera, painful stinging and intraocular inflammation.

The combinations are preferably administered topically. The dosage range for a compound of formula (I) is generally between about 0.01 and about 1000 micrograms per eye (µg/eye) and is preferably between about 0.05 and 5.0 µg/eye. The dosage range for a compound of formula (II) is generally between about 0.001 and about 5.0 µg/eye and is preferably between about 0.01 and 0.5 µg/eye. The combinations of the present invention can be administered as solutions, suspensions, or emulsions (dispersions) in a suitable ophthalmic vehicle.

In forming compositions for topical administration, the mixtures are generally formulated as between about 0.0001 to about 1.0 percent by weight (wt%) solutions in water at a pH between 4.5 to 8.0 (figures relate to combined presence of (I) and (II)). The mixtures are preferably formulated as between about 0.0001 to about 0.1 wt% and, most preferably, about 0.002 wt%. While the precise regimen is left to the discretion of the clinician, it is recommended that the resulting solution be topically applied by placing one drop in each eye two times a day.

Other ingredients which may be desirable to use in the ophthalmic preparations of the present invention include preservatives, co-solvents and viscosity building agents.

Antimicrobial Preservatives:

Ophthalmic products are typically packaged in multidose form. Preservatives are thus required to prevent microbial contamination during use. Suitable preservatives include: benzalkonium chloride, thimerosal, chlorobutanol, methyl paraben, propyl paraben, phenylethyl alcohol, edetate disodium, sorbic acid, Onamer M, or other agents known to those skilled in the art. Such preservatives are typically employed at a level of from 0.001% to 1.0% by weight.

Co-Solvents:

Prostaglandins, and particularly ester derivatives, typically have limited solubility in water and therefore may require a surfactant or other appropriate co-solvent in the composition. Such co-solvents include: Polysorbate 20, 60 and 80; Pluronic F-68, F-84 and P-103; cyclodextrin; or other agents known to those skilled in the art. Such co-solvents are typically employed at a level of from 0.01% to 2% by weight.

Viscosity Agents:

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art. Such agents are typically employed at a level of from 0.01% to 2% by weight.

The following examples are representative pharmaceutical compositions of the invention for topical use in lowering of intraocular pressure. The Compound numbers used in the following Examples refer to the compounds of formula (I) which are listed in Table 1, above.

Example A

| INGREDIENT | PERCENTAGE BY WEIGHT |
|---|---|
| (II): $R'_1 = CH_3$; $R'_2 = H$ | 0.001 |
| (I): Compound 1 | 0.02 |
| Benzalkonium chloride | 0.01 |
| Polysorbate 80 | 0.05 |
| Sodium acetate | 0.07 |
| Sodium chloride | 0.6 |
| Hydroxypropyl methyl cellulose | 0.5 |
| HCl and/or NaOH | to adjust pH |
| Purified water | q.s. to 100% |

Example B

| INGREDIENT | PERCENTAGE BY WEIGHT |
|---|---|
| (II): $R'_1 = CH_3$; $R'_2 = H$ | 0.0005 |
| (I): Compound 2, methyl ester | 0.005 |
| Benzalkonium chloride | 0.01 |
| Pluronic P-84 | 0.5 |
| Dried sodium phosphate | 0.01 |
| Sodium biphosphate | 0.07 |
| Sodium chloride | 0.18 |
| HCl and/or NaOH | to adjust pH |
| Purified water | q.s. to 100% |

Example C

| INGREDIENT | PERCENTAGE BY WEIGHT |
|---|---|
| (II): $R'_1 = CH_3$; $R'_2 = H$ | 0.0002 |
| (I): Compound 3, isopropyl ester | 0.001 |
| Chlorobutanol | 0.5 |
| Sodium acetate | 0.14 |
| Disodium edetate | 0.01 |
| Sodium chloride | 0.52 |
| HCl and/or NaOH | to adjust pH |
| Polyvinyl alcohol | 1.0 |
| Purified water | q.s. to 100% |

Example D

| INGREDIENT | PERCENTAGE BY WEIGHT |
|---|---|
| (II): $R'_1 = CH_3$; $R'_2 = H$ | 0.0002 |
| (I): Compound 3, methyl ester | 0.002 |
| Benzalkonium chloride | 0.01 |
| Dextran 70 | 0.1 |
| Disodium edetate | 0.05 |
| Potassium chloride | 0.12 |
| Sodium chloride | 0.77 |
| Hydroxypropyl methyl cellulose | 0.3 |
| HCl and/or NaOH | adjust pH |
| Purified water | q.s. to 100% |

Example E

| INGREDIENT | PERCENTAGE BY WEIGHT |
|---|---|
| (II): $R'_1 = CH_3$; $R'_2 = H$ | 0.0001 |
| (I): Compound 4, isopropyl ester | 0.001 |
| Benzalkonium chloride | 0.01 |
| Dextran 70 | 0.1 |
| Disodium edetate | 0.05 |
| Potassium chloride | 0.12 |
| Sodium chloride | 0.77 |
| Hydroxypropyl methyl cellulose | 0.3 |
| HCl and/or NaOH | to adjust pH |
| Purified water | q.s. to 100% |

Example F

| INGREDIENT | PERCENTAGE BY WEIGHT |
|---|---|
| (II): $R'_1$ = isobutyl; $R'_2 = H$ | 0.0001 |
| (I): Compound 6, ethyl ester | 0.0005 |
| Benzalkonium chloride | 0.01 |
| Dextran 70 | 0.1 |
| Disodium edetate | 0.05 |
| Potassium chloride | 0.12 |
| Sodium chloride | 0.77 |
| Hydroxypropyl methyl cellulose | 0.3 |
| HCl and/or NaOH | to adjust pH |
| Purified water | q.s. to 100% |

Example G

| INGREDIENT | PERCENTAGE BY WEIGHT |
|---|---|
| (II): $R'_1 = CH_2CH_3$; $R'_2 = H$ | 0.0002 |
| (I): Compound 7, isopropyl ester | 0.001 |
| Benzalkonium chloride | 0.01 |
| Dextran 70 | 0.1 |
| Disodium edetate | 0.05 |
| Potassium chloride | 0.12 |
| Sodium chloride | 0.77 |
| Hydroxypropyl methyl cellulose | 0.3 |
| HCl and/or NaOH | adjust pH |
| Purified water | q.s. to 100% |

Example H

| INGREDIENT | PERCENTAGE BY WEIGHT |
|---|---|
| (II): $R'_1 = CH_3$; $R'_2 = H$ | 0.0001 |
| (I): Compound 8, ethyl ester | 0.005 |
| Benzalkonium chloride | 0.01 |
| Dextran 70 | 0.1 |
| Disodium edetate | 0.05 |
| Potassium chloride | 0.12 |
| Sodium chloride | 0.77 |
| Hydroxypropyl methyl cellulose | 0.3 |
| HCl and/or NaOH | to adjust pH |
| Purified water | q.s. to 100% |

Example J

| INGREDIENT | PERCENTAGE BY WEIGHT |
|---|---|
| (II): $R'_1 = CH_3$; $R'_2 = H$ | 0.001 |
| (I): Compound 5, isopropyl ester | 0.004 |
| Benzalkonium chloride | 0.02 |
| Polysorbate 80 | 0.15 |
| Dibasic sodium phosphate | 0.15 |
| Monobasic sodium phosphate | 0.05 |
| Sodium chloride | 0.75 |
| Disodium EDTA | 0.01 |
| HCl and/or NaOH | to adjust pH |
| Purified water | q.s. to 100% |

The invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims rather than by the foregoing description.

## Claims

**1.** A topical ophthalmic composition for the treatment of glaucoma and ocular hypertension, said composition comprising a combination of:

a) a therapeutically effective amount of a compound of formula (I):

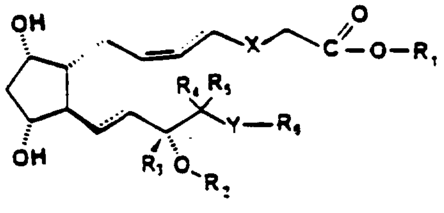

(I)

wherein:

X and Y can be the same or different, and are: $CH_2$ or O;

$R_1$ is hydrogen, a cationic salt moiety, a pharmaceutically acceptable amine moiety or a pharmaceutically acceptable ester moiety derived from the cor-

responding alcohol; and

$R_2$ is hydrogen or a pharmaceutically acceptable ester moiety derived from the corresponding carboxylic acid.

$R_3$, $R_4$ and $R_5$ can be the same or different, and are: H or $CH_3$, with the proviso that if $R_3$ is $CH_3$, then $R_4$ and $R_5$ are H; and

$R_6$ is: $C_{2-7}$ alkyl, thienyl or aryl, optionally substituted with one or more of the following: $C_{1-5}$ alkyl, trifluoromethyl, or a halogen;

with the proviso that if Y is O, and $R_6$ is aryl, then the aryl group must contain at least one substituent; and

b) a therapeutically effective amount of a compound of formula (II):

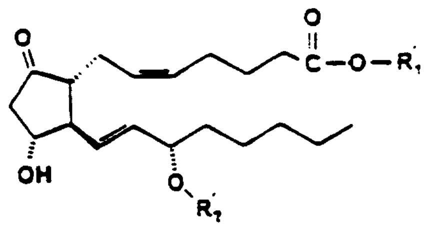

(II)

wherein:

R'$_1$ is hydrogen, a cationic salt moiety, a pharmaceutically acceptable amine moiety or a pharmaceutically acceptable ester moiety derived from the corresponding alcohol; and

R'$_2$ is hydrogen or a pharmaceutically acceptable ester moiety derived from the corresponding carboxylic acid.

**2.** The composition of claim 1, wherein the compound of formula (I) is selected from the group consisting of: cloprostenol, fluprostenol, PhXA41, 16,16-dimethyl-$PGF_{2\alpha}$, 15-methyl-$PGF_{2\alpha}$, 16-(3,5-dichlorophenoxy)-$PGF_{2\alpha}$, tiaprost, 17-phenyl-$PGF_{2\alpha}$, 17-*m*-chlorophenyl-$PGF_{2\alpha}$,17-*m*-trifluoromethylphenyl-$PGF_{2\alpha}$,17-(3,5-dichlorophenyl)-$PGF_{2\alpha}$,and the 3-oxa- and 13,14-dihydro- derivatives thereof.

**3.** The composition of claim 2, wherein the compound of formula (I) is selected from the group consisting of: cloprostenol, fluprostenol and PhXA41.

**4.** The composition of claim 1, wherein between about 0.01 and about 1000 micrograms of a compound of formula (I) is administered.

**5.** The composition of claim 4, wherein between about 0.05 and about 5.0 micrograms of a compound of formula (I) is administered.

**6.** The composition of claim 1, wherein between about 0.001 and about 5.0 micrograms of a compound of formula (III) is administered.

**7.** The composition of claim 6, wherein between about 0.01 and about 0.05 micrograms of a compound of formula (II) is administered.

**8.** The composition of claim 1, wherein the molar ratio of (I):(II) is between about 0.1:1 and about 1000:1.

**9.** The composition of claim 8, wherein the molar ratio of (I):(II) is between about 4:1 and about 20:1.

**10.** The composition of claim 9, wherein the molar ratio of (I):(II) is about 10:1.

**11.** Use of the composition of one or more of claims 1 to 10 for the manufacture of a topical ophthalmic composition for the treatment of glaucoma and ocular hypertension.

European Patent Office

# PARTIAL EUROPEAN SEARCH REPORT

which under Rule 45 of the European Patent Convention shall be considered, for the purposes of subsequent proceedings, as the European search report

Application Number
EP 93 12 0540

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X,<br>Y | US-A-5 173 507 (L. M. DESANTIS) 22 December 1992<br>* the whole document *<br>--- | 1-11 | A61K31/557 |
| Y | EP-A-0 458 587 (K. K. UENO S.) 27 November 1991<br>* claims *<br>--- | 1-11 | |
| Y | EP-A-0 342 003 (K.K UENO S.)<br>* claims *<br>----- | 1-11 | |

TECHNICAL FIELDS SEARCHED (Int.Cl.5)

A61K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 24 February 1994 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 93 12 0540

-C-

INCOMPLETE SEARCH

Claims searched incompletely : 1-11

In view of the definition of products by means of their biological, chemical and or pharmacological properties, the search has to be restricted for economic reasons. The search was limited to the compounds for which pharmacological data was given and/or the compounds mentioned in the claims or examples.
(see guidelines, Part B, Chapter III, paragraph 3.6)